# EUROPÄISCHE PATENTANMELDUNG

(11) **EP 2 008 991 A1**
(43) Veröffentlichungstag der Anmeldung: **31.12.2008**
(21) Anmeldenummer: 07111438.3
(22) Anmeldetag: 29.06.2007
(51) Int. Cl.: C07C 209/68, C07B 37/04

(54) **Verfahren zur Herstellung von Biarylen**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft ein Verfahren zur Herstellung von Biarylen mit Katalysatoren auf Basis von Palladium-Verbindungen mit Phosphanliganden.

## Beschreibung

Die vorliegende Erfindung betrifft ein Verfahren zur Herstellung von Biarylen mit Katalysatoren auf Basis von Palladium-Verbindungen mit Phosphanliganden.

Biaryl-Verbindungen, insbesondere Biphenyl-Verbindungen, haben technische Bedeutung als Feinchemikalien, Zwischenprodukte für Pharmazeutika, optische Aufheller und Agrochemikalien.

Eine häufig angewandte Methode zur Synthese von Biarylen im Labormaßstab ist die Suzuki-Reaktion, bei der Iod- oder Bromaromaten und in Ausnahmefällen Chloraromaten mit Aryl-, Vinyl- oder Alkylboronsäure-Derivaten in Gegenwart von Palladium-Katalysatoren umgesetzt werden. Übersichtsartikel, die diese Methodik beschreiben, findet man beispielsweise in N. Miyaura, A. Suzuki, Chem. Rev. 1995, 95, 2457 und Bellina, F. et al. Synthesis 2004, 2419. Eine Übersicht über die Verwendung von Trialkylphosphinliganden bei der Pd-katalysierten Umsetzung von Chloraromaten findet man in Littke, A.F. & Fu, G.C. Angew. Chem. 2002, 114, 4350.

Katalysatoren, die im Rahmen der Suzuki-Reaktion verwendet werden, sind generell Palladium-und Nickelverbindungen. Trotz des ökonomischen Vorteils von Nickel- Katalysatoren (vgl. A.F. Indolese, Tetrahedron Lett. 1997, 38, 3513) werden aufgrund der geringeren Toxizität und der größeren Toleranz gegenüber funktionellen Gruppen Palladium-Katalysatoren gegenüber NickelKatalysatoren bevorzugt. Im Falle des Einsatzes von Palladium-Katalysatoren werden sowohl Palladium(11)-, als auch Palladium(0)-Komplexe bei Suzuki-Reaktionen eingesetzt (vgl. M. Beller, H. Fischer, W. A. Herrmann, K. Öfele, C. Broßmer, Angew. Chem. 1995, 107, 1992). Als katalytisch aktive Spezies formuliert man gemäß Angaben in der Literatur koordinativ ungesättigte 14- und 16-Elektronen Palladium(0)-Spezies, welche mit Donorliganden wie Phosphanen stabilisiert werden. Insbesondere beim Einsatz von kostengünstigeren Edukten wie Arylbromiden oder Arylchloriden benötigt man den Zusatz von stabilisierenden Liganden, damit eine befriedigende katalytische Aktivierung der Edukte erzielt wird. Ein wesentlicher Nachteil der beschriebenen Suzuki-Reaktionen besteht darin, dass nur mit teuren Ausgangsmaterialien, wie lodaromaten und aktivierten (d. h. elektronenarmen) Bromaromaten, befriedigende katalytische Wechselzahlen ("tumover numbers" = TON) erzielt werden können. Ansonsten müssen bei Verwendung deaktivierter (d. h. elektronenreichen) Bromaromaten oder Chloraromaten große Mengen an Katalysator -üblicherweise 1 bis 5 mol-%- zugesetzt werden, um technisch nutzbare Umsätze zu erzielen.

Darüber hinaus besitzen ortho-substituierte Halogenaromaten aufgrund der größeren sterischen Hinderung eine schlechtere Reaktivität, während Halogen-Aniline problematische Reaktanden sein können, da sie ebenfalls als Liganden für den Katalysator fungieren können.

Die Umsetzung von Fluorhalogenanilinen mit substituierten Boronsäuren in Gegenwart eines Katalysators wird in WO03/070705 beschrieben.

WO 00/61531 beschreibt in diesem Zusammenhang die Verwendung von Katalysatoren mit phosphithaltigen Liganden.

EP 1 186 583 lehrt die Verwendung von Träger-gebundene Pd-Katalysatoren.

EP 1 064 243 und WO 0116057 lehren die Verwendung von allylische Pd-Komplexen, in EP 0 690 046 finden Palladacyclen Einsatz..

Bei all den genannten Verfahren werden teure oder aufwändig herzustellende Palladiumkomplexe eingesetzt oder es ist nötig, zur Erzielung einer guten Ausbeute in Gegenwart eines Überschusses an Arylboronsäure zu arbeiten. Dies erhöht nicht nur die Kosten des Verfahrens durch den Verlust an wertvoller Arylboronsäure, sondern auch durch aufwändigere Reinigungs- und Isolierungsverfahren, die notwendig sind, um überschüssige Boronsäure sowie daraus entstandene Nebenprodukte wie deboronierte Aromaten und Homokupplungsprodukte abzutrennen.

WO 2006/092429 beschreibt die Umsetzung von aromatischen Borinsäuren mit Arylhalogeniden in wässrigen Lösungsmittelsystemen u.a. in Gegenwart von Trialkylphosphinen. Borinsäuren sind jedoch nicht in allen Fällen leicht synthetisch zugänglich.

Der Verlauf der Suzuki-Reaktion wird auch durch die Reaktivität der Boronsäure maßgeblich beeinflusst, wobei insbesondere durch elektronenziehende Substituenten desaktivierte Aromaten langsamer reagieren und Homokupplungsprodukte liefern können. Dieses Problem findet in der methodisch orientierten Literatur jedoch kaum Beachtung, da hier meist in einem großen Überschuss an Boronsäure gearbeitet wird und die Ausbeuten lediglich auf den Umsatz des Halogenaromaten bezogen werden. Ein weiterer Nachteil der im Stand der Technik vorbeschrieben Verfahren ist daher die konkurrierende Homokupplungsreaktion der Halogenaromaten unter Bildung von toxischen polyhalogenierten Biphenylen.

Aufgrund der Komplexität der Reaktionsgemische ist zudem kein einfaches Katalysatorrecycling möglich, so dass auch die Katalysatorkosten in der Regel einer technischen Realisierung entgegenstehen. Katalysatorsysteme auf Basis wasserlöslicher Phosphane ergeben zwar für die industriell bedeutsame Umsetzung von 2-Chlorbenzonitril mit p-Tolylboronsäure befriedigende Katalysatoraktivitäten, jedoch beinhalten die Katalysatoren teure sulfonierte Phosphane.

Aufgabe der vorliegenden Erfindung ist die Bereitstellung eines neuen Verfahrens zur Herstellung von Biarylen, das die Nachteile der bekannten Verfahren nicht aufweist, für die großtechnische Durchführung geeignet ist und Biaryle in hoher Ausbeute und Reinheit bei optimaler Katalysatorproduktivität liefern.

Diese Aufgabe wird gelöst durch ein Verfahren zur Herstellung mono-, bi- und/oder polyfunktioneller Biaryle der allgemeinen Formel (I) 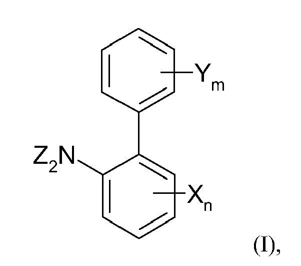 wobei
- Z: Wasserstoff oder Sauerstoff ist
- n: eine ganze Zahl ausgewählt aus 1, 2 oder 3 ist und
- X: unabhängig voneinander ausgewählt sind aus der Gruppe bestehend aus F, Cl, C₁-C₄-Alkyl- und C₁-C₄-Alkyloxy-Gruppen;
- m: eine ganze Zahl ausgewählt aus 0, 1, 2, 3, 4 oder 5 ist und
- Y: unabhängig voneinander ausgewählt sind aus Halogen, C₁₋₄-Alkyl-, C₁₋₄-Alkoxy-, C₁₋₄-Halogenalkyl-, C₁₋₄-Halogenalkoxy-, Hydroxy-Gruppen
durch Umsetzung von Halogenaromaten der allgemeinen Formel (II) 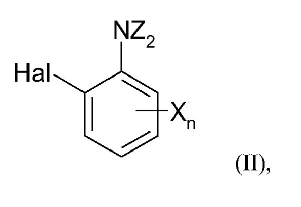 in der
- Hal: ein Halogenatom ist,
mit
(a) wenigstens einer Boronsäure der allgemeinen Formel (III-a) 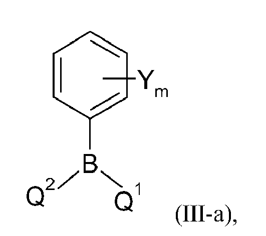 in welcher
   Q¹ und Q² Hydroxyl-Gruppen (-OH) sind
   oder mit den aus den Boronsäuren der Formel (III-a) gebildeten Anhydriden, Dimeren und Trimeren;
   oder mit
   wenigstens einem Boronsäure-Derivat der Formel (III-a),
   in welcher
   - Q¹ und Q²: unabhängig voneinander ausgewählt sind aus der Gruppe bestehend aus F, Cl, Br, I, C₁₋₄-Alkyl-, C₆₋₁₀-Aryl-, C₁₋₄-Alkoxy- und C₆₋₁₀-Aryloxy-Gruppen;
   oder mit
(b) wenigstens einem cyclischen Boronsäureester der Formel (III-b) 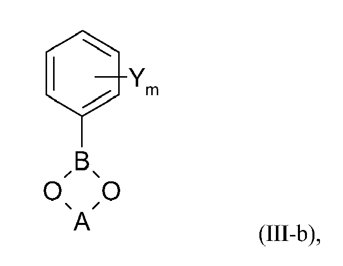 in welcher
   - A: ausgewählt ist aus Resten, die ausgewählt sind aus der Gruppe bestehend aus -CH₂-CH₂-, -C(CH₃)₂-C(CH₃)₂-, -CH₂-C(CH₃)₂-CH₂-;
   oder mit
(c) wenigstens einem Boronat der allgemeinen Formel (III-c) 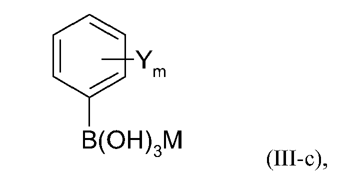
in welcher
- M⁺: ein Kation ist;
in Gegenwart wenigstens eines Palladiumphosphin-Komplexes, wobei die Phosphin-Gruppe mit wenigstens einer verzweigten C₃₋₈-Alkyl-Gruppe substituiert ist.

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare, verzweigte oder ringförmige Kohlenwasserstoff-Gruppen, die optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₁-C₁₂-Alkyl umfasst den größten hierin definierten Bereich für einen AlkylGruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkenyl-Gruppen sind im Zusammenhang mit der vorliegenden Erfindung, soweit nicht abweichend definiert, lineare, verzweigte oder ringförmige Kohlenwasserstoff-Gruppen, die wenigstens eine einfache Unsättigung (Doppelbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkenyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₂-C₁₂-Alkenyl umfasst den größten hierin definierten Bereich für einen AlkenylGruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl, Penta-1,3-dienyl.

Alkinyl-Gruppen sind im Zusammenhang mit der vorliegenden Erfindung, soweit nicht abweichend definiert, lineare, verzweigte oder ringförmige Kohlenwasserstoff-Gruppen, die wenigstens eine zweifache Unsättigung (Dreifachbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkinyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine lineare, verzweigte oder cyclische C₁₋₁₂-Alkyl-Gruppe ist, die ein oder mehrere Heteroatome, ausgewählt aus N, O, P und S, aufweisen kann.

Die Definition C₂-C₁₂-Alkinyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können und optional durch weitere Gruppen substituiert sein können, die ausgewählt sind aus -R', Halogen- (-X), Alkoxy- (-OR'), Thioether-oder Mercapto- (-SR'), Amino- (-NR'₂), Silyl- (-SiR'₃), Carboxyl- (-COOR'), Cyan- (-CN), Acyl-(-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₅₋₁₈-Aryl umfasst den größten hierin definierten Bereich für einen Aryl-Gruppe mit 5 bis 18 Atomen. Im Einzelnen umfasst diese Definition beispielsweise die Bedeutungen Cyclopentadienyl, Phenyl, Cycloheptatrienyl, Cyclooctatetraenyl, Naphthyl und Anthracenyl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether-oder Mercapto- (-SR'), Amino- (-NR'₂), Silyl- (-SiR'₃), Carboxyl- (-COOR'), Cyan- (-CN), Acyl-(-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether-oder Mercapto- (-SR'), Amino- (-NR'₂), Silyl- (-SiR'₃), Carboxyl- (-COOR'), Cyan- (-CN), Acyl-(-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die Alkyl-, Alkenyl-, Alkinyl-, Aryl,- Alkaryl- und Aralkylgruppen können zudem ein oder mehrere Heteroatome aufweisen, die - soweit nicht abweichend definiert - ausgewählt sind aus N, O, P und S. Die Heteroatome ersetzen dabei die bezifferten Kohlenstoffatome. Nicht umfasst sind solche Kombinationen, die den Naturgesetzen widersprechen und die der Fachmann daher, aufgrund seines Fachwissens, ausgeschlossen hätte. Beispielsweise sind Ringstrukturen mit drei oder mehreren benachbarten O-Atomen ausgeschlossen.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Die Halogenaromaten der Formel (II) sind im Zusammenhang mit der vorliegenden Erfindung Fluor-, Chlor-, Brom- oder Iodaromaten. In einer bevorzugten Ausführungsform sind die Halogenaromaten der Formel (II) ausgewählt aus Anilinen (Z=H), besonders bevorzugt ist 2-Brom-4-fluoranilin.

Die Bor-Verbindungen der allgemeinen Formel (III-a) sind vorzugsweise solche, bei denen Q¹ und Q² zusammen mit dem Bor-Atom und zwei Sauerstoffatomen einen fünf oder sechsgliedrigen Ring bilden können, der mit Methyl-Gruppen substituiert sein kann.

Besonders bevorzugt sind Bor-Verbindungen der Formel (III-a) mit Q₁, Q₂= OH, also Boronsäuren der Formel (III-a)) sowie deren Anhydride, Dimere und Trimere.

Weiterhin bevorzugt sind cyclische Boronsäureester der Formel (III-b) 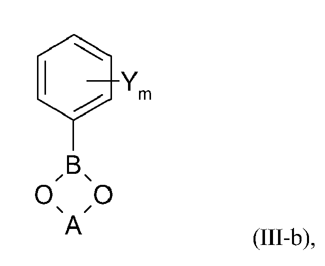 wobei
- A: ausgewählt ist aus der Gruppe bestehend aus -CH₂-CH₂-, -C(CH₃)₂-C(CH₃)₂-, -CH₂-C(CH₃)₂-CH₂-.

Eine ganz besonders bevorzugte Ausführungsform der vorliegenden Erfindung betrifft die Boronsäuren der allgemeinen Formel (III-a) Boronsäuren mit m=2; Y= 3-Cl und 4-Cl, Q₁, Q₂=OH sowie ihre Dimere, Trimere und Anhydride.

Die cyclischen Boronsäureester der allgemeinen Formel (III-b) sind vorzugsweise solche mit Y=Cl und m=2, besonders bevorzugt Y=3-Cl und 4-Cl.

Die Boronate der allgemeinen Formel (III-d) weisen, im Zusammenhang mit der vorliegenden Erfindung, ein Kation (M⁺) auf, welches ausgewählt ist aus Alkali- und Erdalkalimetallen, wie z.B. Li, Na, K, Cs, Mg, Ca und Ba oder aus Tetraalkylammonium-Kationen, wie z.B. NMe₄⁺, NEt₄⁺, NBut₄⁺ oder aus Trialkylammonium-Kationen wie HNEt₃⁺ Vorzugsweise verwendete Boronate der allgemeinen Formel (III-d) sind solche mit Y=Cl, m=2, M⁺ = Na, K, Mg, besonders bevorzugt sind solche mit Y= 3-Cl und 4-Cl.

Die Umsetzung der Borverbindungen findet vorzugsweise in Gegenwart mindestens eines Lösungsmittels statt, welches beispielsweise ausgewählt ist aus der Gruppe bestehend aus Wasser, aliphatischen Ethern, ggfs. halogenierten aromatischen oder aliphatischen Kohlenwasserstoffen, Alkoholen, Estern, aromatischen oder aliphatischen Nitrilen und dipolar aprotischen Lösungsmitteln, wie Dialkylsulfoxiden, N,N-Dialkylamiden aliphatischer Carbonsäuren oder alkylierten Lactamen.

Besonders bevorzugt sind Lösungsmittel, die ausgewählt sind aus der Gruppe bestehend aus THF, Dioxan, Diethylether, Diglyme, Methyl-tert-butylether (MTBE), tert-Amyl-methylether (TAME), Dimethylether (DME), 2-Methyl-THF, Acetonitril, Butyronitril, Toluol, Xylole, Mesitylen, Anisol, Ethylacetat, Isopropylacetat, Methanol, Ethanol, Propanol, Butanol, Ethylenglycol, Ethylencarbonat, Propylencarbonat, N,N-Dimethylacetamid, N,N-Dimethylformamid , N-Methylpyrrolidon, Wasser und Gemischen dieser.

Ganz besonders bevorzugt sind Gemische mit dem umweltfreundlichen Lösungsmittel Wasser.

Es wurde zudem beobachtet, dass der Zusatz geringer Mengen Wasser zu den organischen Lösungsmitteln zu einer weitgehenden Unterdrückung der konkurrierenden Homokupplungsreaktion beiträgt.

Aufgrund der Löslichkeiten der Edukte und der entstehenden Produkte kann jedoch im Allgemeinen nicht gänzlich auf die Anwesenheit eines organischen (unpolaren) Lösungsmittels verzichtet werden. Daher werden die organischen Lösungsmittel vorzugsweise als Cosolvenzien eingesetzt.

Die erfindungsgemäßen Lösungsmittelgemische können zwischen 0,1 und 95 Volumen-% und vorzugsweise zwischen 1 und 60 Volumen-% Wasser, bezogen auf die Mischung aus Wasser und dem organischen Cosolvens, enthalten.

Da bei der Reaktion eine Säure gebildet wird, ist es vorteilhaft, die entstehende Säure durch Zusatz einer Base abzufangen. Die Base kann entweder von Beginn an vorhanden sein oder während der Reaktion kontinuierlich zudosiert werden (semi-batch Verfahren).

Gemäß der vorliegenden Erfindung geeignete Basen sind beispielsweise primäre, sekundäre und tertiäre Amine wie beispielsweise Alkylamine, Dialkylamine, Trialkylamine, die alicyclisch oder offenkettig sein können; Alkali- und Erdalkalisalze aliphatischer und/oder aromatischer Carbonsäuren, wie Acetate, Propionate oder Benzoate; Alkali- und Erdalkali-Carbonate, Hydrogencarbonate, Phosphate, Hydrogenphosphate und/oder Hydroxide; sowie Metallalkoxide, insbesondere Alkali- oder Erdalkalialkoxide, wie beispielsweise Natriummethanolat, Kaliummethanolat, Natriumethanolat, Magnesiummethanolat, Calciumethanolat, Natrium-tert. butylat, Kalium-tert.-butylat oder Alkali-isoamylate. Bevorzugt ist die Base ein Carbonat, Hydroxid oder Phosphat von Lithium, Natrium, Kalium, Calcium, Magnesium oder Cäsium. Besonders bevorzugt sind NaOH, KOH, Pottasche und Soda.

Die eingesetzte Base kann neben der Neutralisation der entstehenden Säure auch durch eine Aktivierung der Arylboronsäure zu anionischen Boranatspezies den Reaktionsverlauf positiv beeinflussen. Neben den oben genannten Basen kann eine solche Aktivierung auch durch Zusatz von Fluoridsalzen wie beispielsweise CaF, NaF, KF, LiF, CsF oder (C,-C,)-Alkyl, NF erreicht werden.

Die eingesetzten Palladiumkatalysatoren werden in der Regel *in situ* aus mindestens einem Palladium(11)salz oder einer Palladium(0)-Verbindung und den entsprechenden Phosphin-Liganden erzeugt. Sie können jedoch auch als Palladium(0)-Verbindung direkt eingesetzt werden, ohne dass dadurch die anfängliche katalytische Aktivität gemindert wird.

Geeignete Palladiumquellen sind beispielsweise ausgewählt aus der Gruppe bestehend aus Palladiumtrifluoracetat, Palladiumfluoracetylacetonat, Pd(OAc)₂, Pd(OCOCH₂CH₃)₂, Pd(OH)₂, PdCl₂, PdBr₂, Pd(acac)₂ (acac = Acetylacetonat), Pd(NO₃)₂, Pd(dba)₂, Pd₂dba₃, (dba = Dibenzyliden-aceton), Pd(CH₃CN)₂Cl₂, Pd(PhCN)₂Cl₂, Li[PdCl₄], Pd/C oder Palladiumnanopartikeln.

Eine bevorzugte Ausführungsform sieht die Verwendung von im Alkyl-Teil verzweigten Methyl-di(C₃₋₈-alkyl)phosphin- oder Tri(C₃₋₈-alkyl)phosphin-Liganden oder deren Salzen, besonders bevorzugt von Methyl-di(tert-butyl)phosphin und Tri(tert-butyl)phosphin als Ligand vor.

Das Trialkylphosphin kann auch als Trialkylphosphonium-Salz wie z.B. als Tetrafluoroborat (Org. Lett. 2001, 3, 4295), Perchlorat oder Hydrogensulfat eingesetzt und hieraus in situ durch Base freigesetzt werden.

Das molare Verhältnis von Palladium zum Phosphin-Liganden sollte zwischen 4:1 und 1:100 liegen, und liegt vorzugsweise zwischen 1 : 1 und 1 : 5, besonders bevorzugt zwischen 1 : 1 und 1 : 2.

Erfindungsgemäß kann aber auch direkt Pd[P(t-But)₃]₂ verwendet werden, dessen Herstellung in (J. Amer. Chem. Soc. 1976, 98, 5850; J. Amer. Chem. Soc. 1977, 99, 2134; J. Am. Chem. Soc. 2001, 123, 2719) beschrieben ist.

Eine weitere bevorzugte Ausführungsform beinhaltet die Verwendung von 1,1-Bis-(di-t-butylphosphino)-ferrocen (D.t.BPF) als Ligand am Palladium.

Bei der Durchführung der Reaktion kann das Katalysatorsystem (Pd + Ligand) zusammen oder getrennt entweder bei Raumtemperatur oder in der Wärme zugegeben werden. Man kann das System kurz vor der Durchführung separat durch Zusammengeben eine Pd-Salzes und des Liganden herstellen oder in kristalliner Form käuflich erwerben. Man kann auch direkt in den Ansatz erst den Liganden und anschließend das Palladiumsalz hinzufügen (*in situ* Verfahren).

Gemäß der vorliegenden Erfindung werden die Halogenaromaten der Formel (II) und die Bor-Verbindungen der Formeln (III-a) bis (III-c) in einem äquimolaren Verhältnis eingesetzt. Alternativ kann jedoch auch eine der beiden Komponenten (II oder III), vorzugsweise die Bor-Verbindungen (III-a) bis (III-c), im Überschuss eingesetzt werden. Es ist auch möglich, die Reaktion dosierkontrolliert durchzuführen, wobei eine der beiden Reaktionskomponenten während der Reaktion langsam zudosiert wird. Bevorzugt verwendet man hierfür z.B. eine Lösung der Boronsäure oder des Boronats, während die Halogenkomponente, der Katalysator und ggf. die Base vorgelegt werden.

Die Umsetzung wird im allgemeinen bei einer Temperatur zwischen 10 und 200° C, vorzugsweise zwischen 20 und 140°C, sowie bei einem Druck bis zu 100 bar, vorzugsweise bei einem Druck zwischen Normaldruck und 40 bar, durchgeführt.

Die Reaktion erfolgt vorzugsweise unter Ausschluss von Luftsauerstoff unter Schutzgasatmosphäre, wie z.B. unter Argon- oder Stickstoffatmosphäre.

Aufgrund der Katalysatoraktivitäten und -stabilitäten ist es mit dem erfindungs- gemäßen Verfahren möglich, extrem kleine Mengen an Katalysator zu verwenden, so dass die Katalysatorkosten im Vergleich zu den bekannten Suzuki-Reaktionen für den entsprechenden Prozess nicht limitierend sind.

Bei dem erfindungsgemäßen Verfahren werden Gehalte an Katalysatoren von 0,0001 bis 5 mol-%, besonders bevorzugt < 0,1 mol-%, bezogen auf die Halogenkomponente, verwendet.

Aufgrund der geringen Katalysatormengen kann der Katalysator in den meisten Fällen im Endprodukt verbleiben. Alternativ kann jedoch auch eine Aufreinigung der erhaltenen Biaryle durch Filtration z.B. über Celite erfolgen.

Die nachstehenden Beispiele dienen der Erläuterung des erfindungsmäßen Verfahrens, ohne es darauf zu beschränken.

### Beispiele für die Darstellung von 3',4'-dichlor-5-fluorbiphenyl-2-amin

Die Beispiele belegen, dass es bei dem erfindungsgemäßen Verfahren möglich ist, mit weniger als 0,1 mol-% Katalysator- und Ligandmenge hohe Ausbeuten zu erzielen und dabei nur sehr geringe Mengen an (<1 % anstelle von z.B. 10%) Homokupplungsprodukten der Boronsäure zu erzeugen.Mit *kommerziellem Katalysator (0.01 mol%) in Acetonitril- Wasser*

Man gibt 29,5 g (213.5 mmol) Pottasche in 210 ml Wasser, gibt 22.3 g (94,2 %ig, 110.1 mmol) 3,4-Dichlorphenylboronsäure (enthielt 1% 3,4-Dichlorbrombenzol und 0.3% PCB077) und 150 ml Acetonitril hinzu, rührt 25 Minuten lang und gibt 19.16 g (99.2%ig, 100 mmol) 2-Brom-4-fluoranilin in 50 ml Acetontril hinzu. Die Lösung wird sechsmal evakuiert und mit Argon begast und anschliessend mit 6 mg Bis(tri-t-butylphosphin)palladium versetzt. Man rührt unter Argon bei 67-69°C 20 Stunden lang, lässt abkühlen, gibt 150 ml Essigsäureethylester hinzu, trennt die organische Phase ab, extrahiert noch zweimal mit je 50 ml Essigsäureethylester und erhält nach dem Eindampfen der vereinigten organischen Phasen 29,25 g eines kristallisierenden Öls. Reinheit (HPLC) 86,2 %; Ausbeute 99.1 %.

### Mit frisch hergestelltem Katalysator (0. 01 mol%)

548 mg einer 12.9%igen Lösung von Tri-t-butylphosphin in Toluol wird in 20 ml THF gelöst. 2.28 ml dieser Lösung werden mit 4 ml einer Lösung von 69 mg Pd2dba3 in 15 ml THF versetzt und 10 Minuten gerührt. Eine Argon-gesättigte Lösung aus 19.35 g (99.2%ig, 100 mmol) 2-Brom-4-fluoranilin, 22.5 g (94,2 %ig, 110.1 mmol) 3,4-Dichlorphenylboronsäure und 29.3 g (212 mmol) Pottasche in 115 ml Wasser und 115 ml Toluol wird mit 1.57 ml obiger Katalysator-Lösung versetzt und bei 67-69°C 17 h gerührt. Die organische Phase wird abgetrennt und die wässrige Phase einmal mit 50 ml Toluol gewaschen. die vereinigten organischen Phasen werden im Vakuum eingedampft, wobei 27.93 g eines kristallisierenden Öles zurückbleiben. Reinheit (GCMS): 89%. Ausbeute:89.8 %. PCB <0.1 %

### Mit 1, 1-Bis-(di-t-butylphosphino)-ferrocen (D.t.BPF)

### in Toluol/Wasser

8,15 g (97%ig, 0,042 mol) 3,4-Dichlorphenylboronsäure werden in 50 g Wasser und 50 g Toluol vorgelegt. Anschließend werden 18,7 g (0,085 mol) Kaliumphosphat und 8,2 g (98%ig, 0,042 mol) 2-Brom4-fluoranilin zugegeben. Nach Inertisieren mit Stickstoff werden 0,014 g (0,00002 mol) 1,1'-Bis(di-tert.-butylphosphino)ferrocen-palladiumdichlorid zugegeben und das Gemisch zwei Stunden bei 79-81°C gerührt. Die organische Phase wird abgetrennt und im Vakuum eingedampft, wobei 10,5 g eines kristallisierenden Öles zurückbleiben. Reinheit (HPLC): 97%, Ausbeute: 95%. PCB <1%

### in THF/Wasser

8,15 g (98%ig, 0,042 mol) 2-Brom-4-fluoranilin und 7,8 g (97%ig, 0,040 mol) 3,4-Dichlorphenylboronsäure werden in 20 g Wasser und 50 g Tetrahydrofuran vorgelegt. Nach Inertisieren mit Stickstoff werden anschließend 0,014 g (0,00002 mol) 1,1'-Bis(di-tert.-butylphosphino)ferrocen-palladiumchlorid zugegeben und das Gemisch auf 65-67°C erhitzt. Dann wird innerhalb von einer Stunde eine Lösung von 13,4 g (99,8%ig, 0,1262 mol) Natriumcarbonat in 30 g Wasser zugetropft und nach beendeter Dosierung zwei Stunden bei 65-67°C nachgerührt. Die organische Phase wird abgetrennt und im Vakuum eingedampft, wobei 10,5 g eines kristallisierenden Öles zurückbleiben. Reinheit (HPLC): 96%, Ausbeute: 93,7%. PCB <1%.*semi-batch in Toluol*/*Wasser mit NaOH*

85,46 g (99.2%ig, 0,446 mol) 2-Brom-4-fluoranilin und 90,2 g (0,473 mol) 3,4-Dichlorphenylboronsäure werden in 200 g Wasser und 565 g Toluol vorgelegt. Nach Inertisieren mit Stickstoff wird auf 85°C erhitzt und 25.9 mg (0.02 mol%) Tri-tert.-butylphosphin tetrafluoroborat in 5 ml Wasser und 27.2 mg (0.02 mol%) Palladium(II)acetylacetonat in 5 ml Toluol zugegeben.

Dann wird innerhalb von etwa zwei Stunden eine 10proz. Natronlauge so zugetropft, das ein pH-Wert von 8 - 8,5 gehalten wird. Hierzu werden etwa 1.2-1.4 Äquivalente benötigt. Nachdem mit HPLC ein kompletter Umsatz festgestellt wird, wird die organische Phase abgetrennt und im Vakuum eingedampft, wobei 10,5 g eines kristallisierenden Öles zurückbleiben. Reinheit (HPLC): 96%, Ausbeute: 93,7%. PCB <1%. Zur weiteren Aufreinigung wird mit konz. HCl gefällt, das ausgefallene Hydrochlorid mit Toluol gewaschen und mit Toluol/MeOH/Wasser/NaOH freigesetzt. Aus der organischen Phase erhält man durch Eindampfen im Vakuum das Zielprodukt als Öl (157.9 g; Gehalt (HPLC gegen Standard): 71,2 %; Ausbeute 89.5 % .

## Patentansprüche

1. Verfahren zur Herstellung mono-, bi- und/oder polyfunktioneller Biaryle der allgemeinen Formel (I) 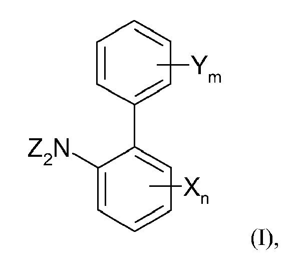 wobei
Z Wasserstoff oder Sauerstoff ist
n eine ganze Zahl ausgewählt aus 1, 2 oder 3 ist und
X unabhängig voneinander ausgewählt sind aus der Gruppe bestehend aus F, Cl, C₁-C₄-Alkyl- und C₁-C₄-Alkyloxy-Gruppen;
m eine ganze Zahl ausgewählt aus 0, 1, 2, 3, 4 oder 5 ist und
Y unabhängig voneinander ausgewählt sind aus Halogen, C₁₋₄-Alkyl-, C₁₋₄-Alkoxy-, C₁₋₄-Halogenalkyl-, C₁₋₄-Halogenalkoxy-, Hydroxy-Gruppen
durch Umsetzung von Halogenaromaten der allgemeinen Formel (II) 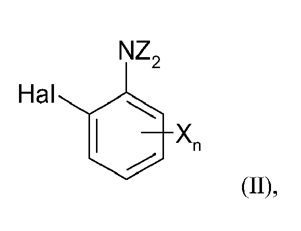 wobei
Hal ein Halogenatom ist,
mit
(a) wenigstens einer Boronsäure der allgemeinen Formel (III-a) 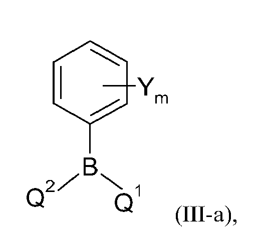 in welcher
Q¹ und Q² Hydroxyl-Gruppen (-OH) sind
oder mit den aus den Boronsäuren der Formel (III-a) gebildeten Anhydriden, Dimeren und Trimeren;
oder mit wenigstens einem Boronsäure-Derivat der Formel (III-a),
in welcher
Q¹ und Q² unabhängig voneinander ausgewählt sind aus der Gruppe bestehend aus F, Cl, Br, I, C₁₋₄-Alkyl-, C₆₋₁₀-Aryl-, C₁₋₄-Alkoxy- und C₆₋₁₀-Aryloxy-Gruppen;
oder mit
(b) wenigstens einem cyclischen Boronsäureester der Formel (III-b) 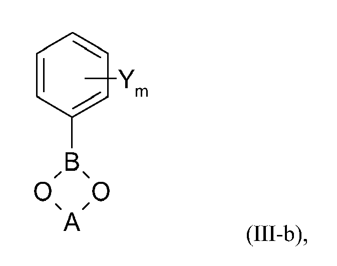 in welcher
A ausgewählt ist aus Resten, die ausgewählt sind aus der Gruppe bestehend aus - CH₂-CH₂-, -C(CH₃)₂-C(CH₃)₂-, -CH₂-C(CH₃)₂-CH₂-;
oder mit
(c) wenigstens einem Boronat der allgemeinen Formel (III-c) 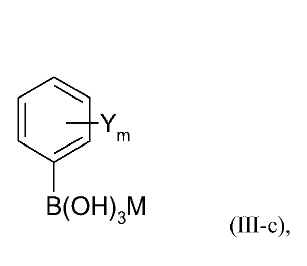
in welcher
M⁺ ein Kation ist;
in Gegenwart wenigstens eines Palladiumphosphin-Komplexes, wobei die Phosphin-Gruppe mit wenigstens einer verzweigten C₃₋₈-Alkyl-Gruppe substituiert ist.

2. Verfahren gemäß Anspruch 1, wobei die Umsetzung in Gegenwart eines organischen Lösungsmittels erfolgt.

3. Verfahren gemäß Anspruch 2, wobei das Lösungsmittel zwischen 0,1 und 95 Volumen-% Wasser, bezogen auf die Mischung aus Wasser und dem organischen Cosolvens, enthält.

4. Verfahren gemäß einem der Ansprüche 1 oder 2, wobei der Halogenaromat der Formel (II) 2-Brom-4-fluoranilin ist.

5. Verfahren gemäß einem der Ansprüche 1 bis 3, wobei der Palladiumkomplex ausgewählt ist aus Bis(tri-*tert*-butylphosphin)palladium, Bis[methyl-di(tert-butyl)phosphin]palladium und [1,1-Bis-(di-t-butylphosphino)-ferrocen]palladium.

6. Verfahren gemäß einem der Ansprüche 1 bis 4, wobei der Palladiumkomplex *in situ* durch Zugabe einer Palladium-Quelle und eines Pd-Liganden erzeugt wird.

7. Verfahren gemäß Anspruch 5, wobei der Ligand Tri(tert-butyl)phosphin oder eines seiner Salze ist.

8. Verfahren gemäß Anspruch 5, wobei der Ligand Methyl-di(tert-butyl)phosphin oder eines seiner Salze ist.

9. Verfahren gemäß einem der Ansprüche 6 bis 7, wobei das jeweilige Hydrogen-Tetrafluoroborat-Salz (HBF4-Salz) verwendet wird.

10. Verfahren gemäß einem der Ansprüche 5 bis 8, wobei die Palladium-Quelle Palladiumacetylacetonat oder Palladium-dibenzylidenacetonat ist.

11. Verfahren gemäß Anspruch 1, wobei die Boronsäure der Formel 3,4-Dichlorphenylboronsäure ist.
